# EUROPEAN PATENT SPECIFICATION

(11) **EP 1 682 218 B1**
(45) Date of publication and mention of the grant of the patent: **10.12.2008**
(21) Application number: 04791157.3
(22) Date of filing: 05.10.2004
(51) Int. Cl.: A61N 1/36

(54) **IMPROVED CEREBRAL ELECTROSTIMULATION DEVICE**
VERBESSERTE ZEREBRALE ELEKTROSTIMULATIONSVORRICHTUNG
APPAREIL D'ELECTROSTIMULATION CEREBRALE PERFECTIONNE

(30) Priority: 14.10.2003 FR 0350675
(43) Date of publication of application: 26.07.2006
(73) Proprietor: COMMISSARIAT A L'ENERGIE ATOMIQUE, 75015 Paris (FR); INSTITUT NATIONAL DE LA SANTE ET DE LA RECHERCHE MEDICALE, 75654 Paris Cedex 13 (FR)
(72) Inventor: CAILLAT, Patrice, F-38130 Echirolles (FR); BOURGERETTE, Alain, F-38190 VILLARD-BONNOT (FR); VACHERAND, François, F-38800 PONT-DE-CLAIX (FR); BENABID, Alim-Louis, F-38240 MEYLAN (FR)
(74) Representative: Poulin, Gérard
(86) International application number: PCT/EP2004/052446
(87) International publication number: WO 2005/039694

(56) References cited:
- WO-A-03/014789
- WO-A-20/04096348
- US-A- 3 311 111
- US-A1- 2002 077 670
- US-A1- 2003 125 786
- US-B1- 6 456 190

## Description

### TECHNICAL DOMAIN AND PRIOR ART

The invention relates to the domain of cerebral electrostimulation.

It proposes an improved cerebral electrostimulation device that can for example be used for the treatment or study of cerebral pathologies such as epilepsy or Parkinson's disease.

A cerebral electrostimulation method used in prior art to electrically stimulate a target region of a patient's brain, consists firstly of precisely locating the target region to be stimulated in the brain using one or more measurement electrodes in contact with the brain. The measurement electrodes are introduced inside guides passing through openings made in the patient's skull and put into contact with the brain. They are then used to make an electrical reading of the patient's cerebral activity, for example when the patient moves.

Electrodes are tested in turn so as to determine the measurement electrode closest to the target region to be stimulated, based on the electrical reading of the patient's cerebral activity.

This phase to locate the target region to be stimulated is usually long and can take several hours in the operating theatre.

The entire operation lasts for about 6 to 10 hours.

Once the measurement electrode closest to the target region has been determined, all measurement electrodes are withdrawn from the guides and a stimulation electrode is then placed in the guide that contained said measurement electrode closest to the target region that had been located.

The stimulation electrode may for example comprise four active areas at a spacing from each other. It is connected through a connector to a stimulator with 4 channels, each of which can send electrical pulses. Each stimulator channel is connected to an active area of the electrode. Thus, an electrical pulse is sent on an active electrode area by energising the corresponding stimulator channel.

For example, if it is required to change the target region to be stimulated, or if the stimulation electrode moves and it no longer stimulates the target region and instead it stimulates another region of the brain, the method described above is usually applied entirely in the operating theatre, starting from detection of the target region and continuing until implantation of the stimulation electrode.

Therefore, the problem arises of finding a new device capable of minimising the time spent in the operating theatre.

Document US 2002/0077670 discloses a cerebral electrostimulation device according to the preamble of claim 1.

### PRESENTATION OF THE INVENTION

This invention relates to a cerebral electrostimulation device as defined in the accompanying claims.

A biocompatible electrode means an electrode made of a material that can be present in brain tissues.

The connector is used to vary the cerebral stimulation area after the electrode or electrodes has (have) been implanted without it being necessary to keep the patient in the operating theatre.

For example, the switching means may comprise switches; they may also comprise several inputs connected to one or several channels of a stimulator or stimulating means.

A cerebral electrostimulation device according to the invention may also comprise stimulating means or at least one stimulator, comprising one or several channels connected to one or several inputs of the commutation device, so that electrical pulses can be sent to a patient's brain.

According to another variant, the cerebral electrostimulation device according to the invention may include a device or measurement means with one or several channels connected to one or several inputs of the commutation device.

It is then possible to make cerebral activity measurements.

For example, the electrostimulation device according to the invention can be used to connect one channel of a stimulator emitting stimulation signals to one or several active electrode areas, or to one or several active areas on different electrodes, or to all active areas of electrodes in the device.

Thus, with a single channel of a stimulator or a measurement device, and one or several electrodes, the range and position of areas to be stimulated or measured can be varied using the electrodes.

The device according to the invention can also be used to change from a measurement phase, for example for measuring a cerebral activity, to a phase to stimulate a region of a brain, if the commutation device is connected to a combination of measurement electrodes and stimulation electrodes, or if it is connected to hybrid electrodes so that a measurement and a stimulation can be made.

According to one variant, the commutation device may be implanted subcutaneously. The electrodes are generally implanted in a patient's skull, and the commutation device can then be placed close to the electrodes.

In one arrangement, not according to the invention, the switching means may be semi-conductor switches. According to the invention, the switching means are electromechanical bistable switches included in a micro electromechanical system (MEMS).

Electromechanical switches or micro-electromechanical switches enable the commutation device to consume less energy, particularly compared with conventional switches. Electromechanical bistable switches or micro-electromechanical bistable switches, for example made in a MEMS, consume very little energy when they are in a given state, ("open" or "closed"). The greatest energy consumption is necessary for a state change. If the device according to the invention is used in an application in which the switches do not change state very often, electromechanical bistable switches can be used to make a commutation device with very low consumption.

Switches can also be arranged in a matrix form. This type of switch matrix can be used to connect each input of the commutation device with any of its outputs, or with several of its outputs.

The cerebral electrostimulation device according to the invention may include a control device external to the commutation device capable of controlling or programming the commutation device, or a circuit included in this device, by radio and / or electrical signals.

The control device, for example using radio frequency or electrical configuration signals, may then be used to activate opening or closing of each switch in the commutation device.

The control device may include at least one first remote transmission module, for example provided with one or several antennas capable of sending radio configuration signals in order to control or program the commutation device.

According to one variant, the control device may also be used to send radio-frequency power supply signals to at least partly supply power to the commutation device. The control device is then used to send these radio power supply signals through said transmission module or a second remote transmission module.

The commutation device may also comprise one or several antennas, for example to pick up power supply signals or radio frequency configuration signals from said remote transmission module of the control device.

Programming means, for example a computer provided with a programming interface, may be provided to program the control device.

The cerebral electrostimulation device may also comprise power supply means for supplying power to the commutation device. These power supply means may include a power supply integrated in the commutation device and / or a power supply integrated into the stimulator.

The power supply means may also comprise a remote power supply device, in other words a device with an energy source external to the commutation device, capable of supplying energy to the commutation device for example in the form of radio waves. The remote power supply device may also include energy collection means integrated into the commutation device capable of picking up said energy.

According to one variant of the device according to the invention, the commutation device is subcutaneous and can also be used to selectively connect each of its inputs to a single output.

A treatment process for a disease such as Parkinson's disease or epilepsy may advantageously be made using a device according to the invention; electrodes and a commutation device like that described above are implanted in a patient's brain, and a stimulator sends pulses that are transmitted to the electrodes selected by the commutation device.

The configuration of the commutation device may easily be programmed or modified without the need for a surgical operation.

This type of operation is only carried out during implantation of the electrodes and the commutation device in the patient's brain, while the test phases to determine the electrodes or areas to be activated, or to correctly program the commutation device, are done after the operation outside the operating theatre. Therefore, this process is much more economic than known techniques that require the operating theatre to be immobilised both during the test phases and the implantation, phases of the stimulation electrodes.

Therefore, the invention enables implantation of electrostimulation electrodes before precise localisation of areas to be activated in the brain.

### BRIEF DESCRIPTION OF THE FIGURES

This invention will be better understood after reading the description of example embodiments given purely for information purposes and which are in no way limitative, with reference to the appended figures, wherein:
- figures 1-7 represent different variants of electrostimulation devices according to the invention,
- figures 8A and 8B illustrate examples of therapeutic methods using the electrostimulation device according to the invention,
- figure 8C represents a variant of the electrostimulation device according to the invention.

Identical, similar or equivalent parts in the different figures are not necessarily shown with the same references, to make the figures more easily comparable.

The different parts shown on the figures are not necessarily all shown at the same scale, to make the figures more easily understandable.

### DETAILED PRESENTATION OF PARTICULAR EMBODIMENTS

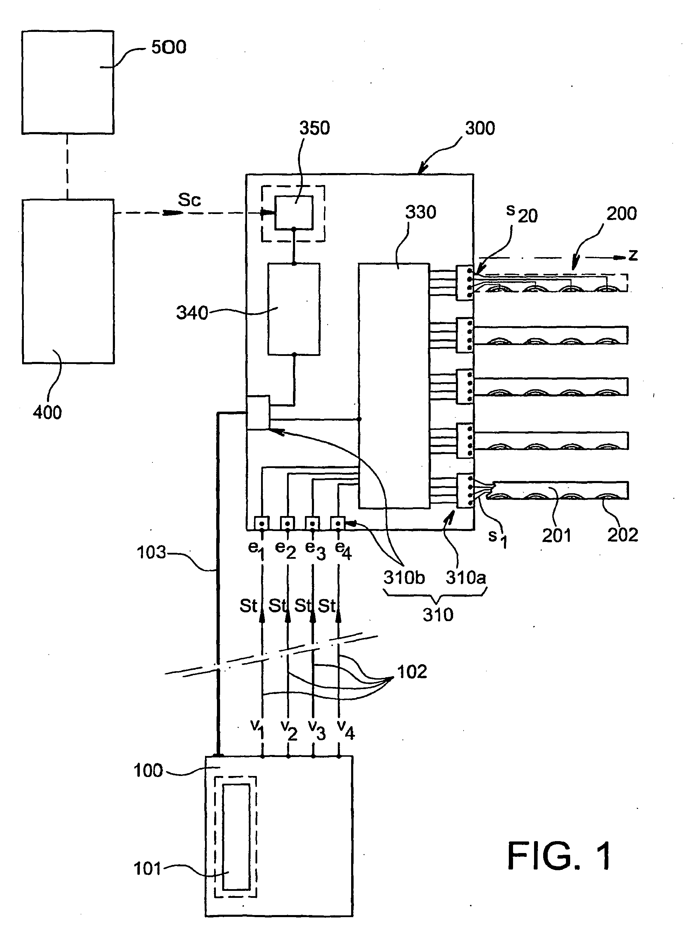

One example of an electrostimulation device according to this invention and comprising at least one commutation device 300, will now be described with reference to figure 1, with the environment consisting of a stimulator 100 and a control device 400.

A stimulator 100 comprises 4 channels v1, ..., v4, from which it can send one or several stimulation signals St, preferably continuously, in the form of electrical pulses on one or more of its 4 channels v1, v..., v4.

The amplitude of the electrical pulses may be a few volts, for example between 1 and 10 volts. The frequency of the pulses is preferably between 1 Hz and 10 Hz, or between 10 and 20 kHz. The pulses may be sent at a frequency, for example, of the order of a hundred hertz, for example between 100 Hz and 150 Hz. For example, the pulse width may vary between 60 µs and 120 µs.

These electrical pulses will be sent to electrodes 200 implanted in the patient's brain. The stimulator 100 may or may not be implanted subcutaneously. It comprises a power supply denoted 101, for example a battery.

The number of stimulator channels included in the device according to the invention is not limited to 4, it may be more than or less than 4. Furthermore, the stimulator can send electrical pulses on only one of its channels, or on several of its channels, or on all its channels. For example, it sends electrical pulses with different amplitudes and / or different frequencies and / or different widths on each of its channels v1, ..., v4.

The electrodes 200 (there are 5 shown in figure 1) are to be implanted in the patient's skull close to a target region to be stimulated, for example a region close to the subthalamic nucleus of the patient.

There may be several types of electrodes 200. There may be stimulation electrodes, capable of applying electrical pulses for example to stimulate the target region of the patient's brain. There may also be hybrid electrodes capable of applying electrical pulses, and also capable of making electrical measurements corresponding to a cerebral activity. Finally some electrodes 200 may be measurement electrodes, dedicated solely to cerebral activity measurements.

The example device illustrated in figure 1 may comprise stimulation electrodes only, or hybrid electrodes only, or a combination of electrodes with at least one stimulation electrode and / or at least one hybrid electrode, and possibly one or several measurement electrodes.

In the example device illustrated in figure 1, each electrode is in the form of a tube 201 extending along a z axis. The tube 201 surrounds 4 rods each at a spacing from the others. These rods are each made from a conducting material such as a noble metal or a non-oxidisable metal, preferably a biocompatible metal, so that the electrodes will be tolerated by the organism in which they will be implanted. The 4 rods terminate in 4 end piece each arriving at different levels along the tube 201, along the z axis. Each end pieces terminates at openings along the tube. Each forms an active area 202. The active areas 202 are areas in which electrical pulses are applied and may come into contact with a patient's brain. There were 5 electrodes in the example device illustrated in figure 1, the device comprises a total of 20 active areas 202 that may carry electrical signals.

In the same way as the rods, the electrode tube is preferably made from a biocompatible material or it comprises a biocompatible material, for example such as silicone.

The number of active areas per electrode is not limited to 4, the electrodes included in the device according to the invention may have one or several active areas. Nor is the number of electrodes included in the device according to the invention limited to 5. The device according to the invention may comprise more or less than 5 electrodes.

The stimulator 100 and the electrodes 200 are connected together through a commutation device 300.

This commutation device may or may not be implanted subcutaneously.

If it is designed to be implanted subcutaneously, it will preferably be embedded in a biocompatible material such as silicone. The commutation device 300 will preferably be implanted in a region close to the target region to be stimulated in a patient's brain.

The commutation device 300 comprises connectors 310, some of which are output connectors 310b and dedicated to the connection of electrodes 200. Others are input connectors 310a and are used for the input of links, some of which, denoted 102, are vectors of stimulation signals or electrical pulses from the 4 channels v1, ..., v4 of the stimulator 100, the reference 103 designating a power supply cable connected to a stimulator power supply 101.

The commutation device 300 is powered by power supply means internal to it or through the power supply 101 of the stimulator 100.

The commutation device 300 illustrated in figure 1 comprises 4 inputs denoted e1, ..., e4, each connected to a channel of the stimulator 100. The commutation device 300 comprises 20 outputs denoted s1, ... s20, each connected to one of the 20 active areas 202 of the electrodes 200, and it is used to select one or several of its inputs and to connect each of the selected inputs to one or several outputs that are also selected. Thus, the commutation device can be used to carry electrical pulses from one of its inputs to one or several outputs, or even to all of its outputs, in other words to one or several active electrode areas, or to all active electrode areas.

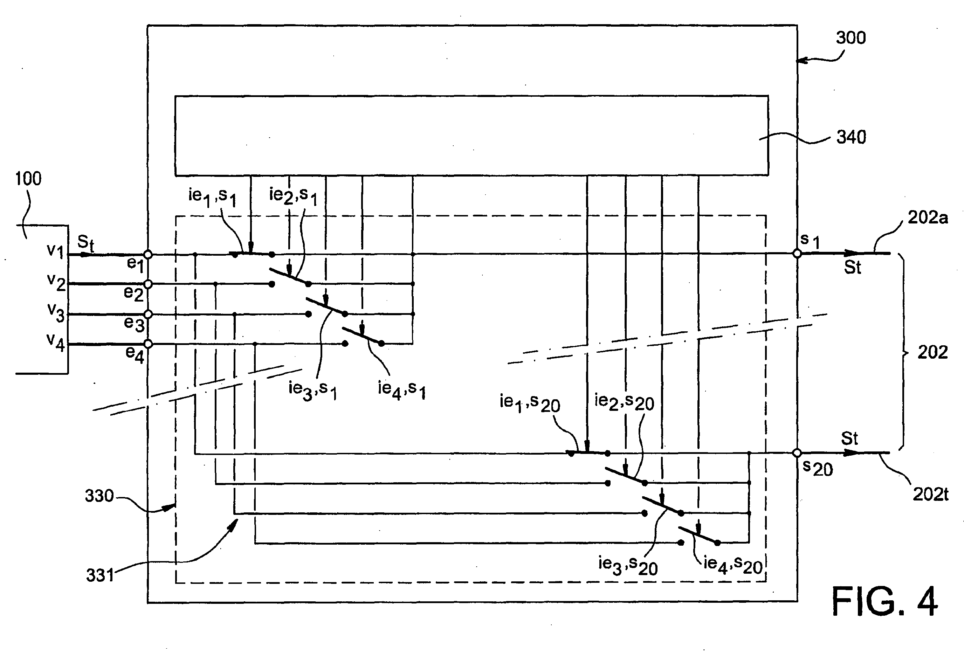

The commutation device 300 may comprise an electronic or electromechanical switching circuit 330, that directs stimulation signals originating from at least one input of the device 300, to one or several outputs of this same device 300. It is provided with means performing the function of switches (not shown in figure 1) which, depending on their state (open or closed), control routing between the inputs e1, ..., e4 and the outputs s1, ..., s20. Thus, the switching means may be arranged in a matrix form as in the example device illustrated in figure 4 described below.

The switches are electromechanical bistable switches. In this case, opening or closing is triggered mechanically, the mechanical action usually being triggered by an electrical excitation or by a surface acoustic wave or by a large and preferably very local temperature increase, which are themselves triggered by an electrical signal.

Thus, the switching circuit 330 comprises a MEMS (micro-electromechanical system) provided with switches performing an electromechanical bistable switches function and usually only requiring a very small amount of energy to remain in a stable state (open or closed). Consumption of the commutation device is then very low when the switches remain in a stable state, which is very attractive in the case of a subcutaneous implantation.

The state change of each switch in the switching circuit 330 may be triggered by a control circuit 340 included in the commutation device 300.

If the switching circuit 330 is electronic, the control circuit keeps the switches in the "open" or "closed" state, and can also trigger a state change.

If the switching circuit 330 comprise a MEMS or if the switches are micro-electromechanical bistable switches, the control circuit 340 can send one or several electrical signals to the switching circuit 330 that the switching circuit 330 can convert, for example, to a mechanical action to open or close one or more switches.

The control circuit 340 itself can be programmed or controlled by a control device 400 external to the patient.

This control device 400 can transmit a configuration program or order to the commutation device 330 in the form of electrical or radio frequency configuration signals Sc to control it or program it, for example to inform it that it should change the state of one or several or all of its switches by opening or closing them.

The commutation device 300 can contain the configuration program or order in the form of configuration signals Sc by signal receptor means 350.

The device illustrated in figure 1 also comprises programming means 500, for example a computer provided with a programming interface, capable of generating the configuration program or order, and transferring it to the control device 400, which may be a peripheral of the programming means.

The transfer operation to the control device is usually done under the control of the doctor; this is the operation by which he will control or program the commutation device.

According to one variant not shown, the control device 400 may be connected to the commutation device through one or several cables, so that the configuration program or order can transit in the form of electrical configuration signals Sc. The signal reception means 350 of the commutation device 300 then comprise connectors to attach the cables to the commutation device 300.

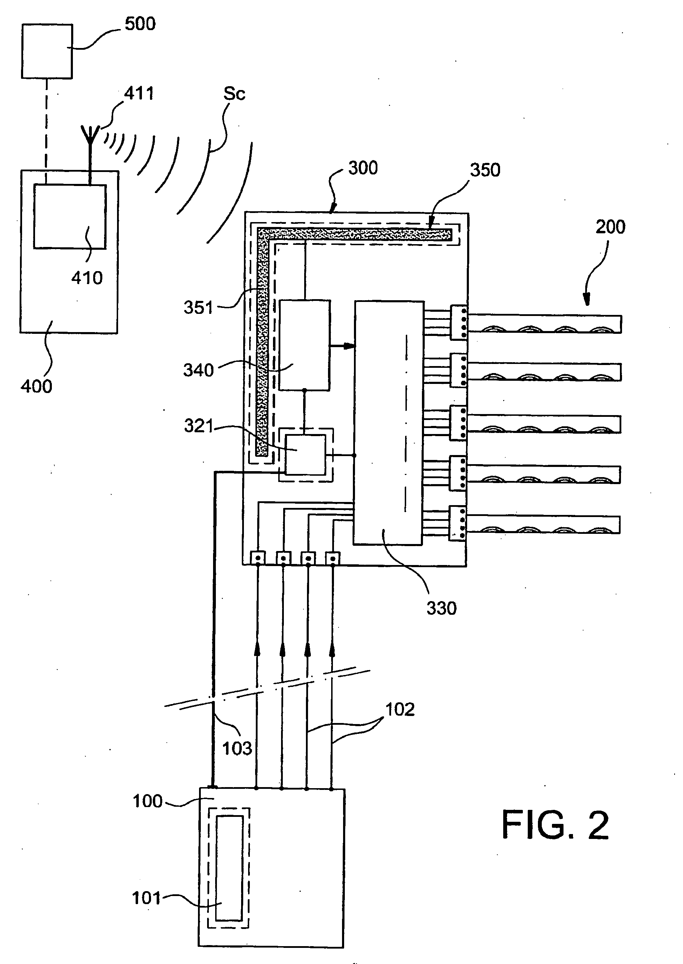

Figure 2 illustrates a variant of the device according to the invention that is different from the device shown in figure 1, in that the control device 400 is capable of transmitting a configuration program or order in the form of radio frequency configuration signals Sc, through a wireless link. The control device 400 comprises a remote transmission module 410 provided with an antenna 411 capable of sending configuration signals Sc in the form of radio frequency signals. The signal reception means 350 at the commutation device end are provided with another antenna 351 capable of picking up the radio configuration signals Sc.

Figure 2 is also different from figure 1 in that the power supply means comprise a power supply 321 integrated into the commutation device 300. This integrated power supply 321 is capable of at least partially supplying power to the commutation device 300. It can supply power to the commutation device 300 alone, or it can operate in cooperation with other power supplies such as power supply 101 of the stimulator (figure 1) and / or a remote power supply device.

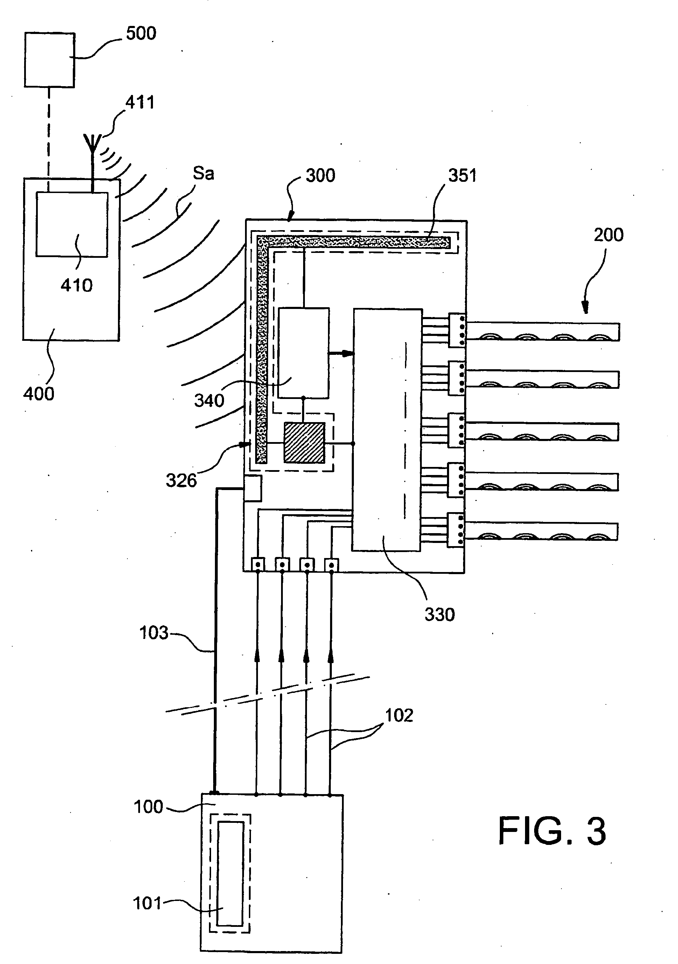

Figure 3 illustrates another example device according to the invention that is different from that shown in figure 2 in that the power supply means comprise a remote power supply device. The commutation device 300 illustrated in figure 3 comprises energy collection means 326 capable of picking up energy supplied by a radio wave originating from an energy source external to the commutation device 300. For example, the energy source may originate from the control device 400 and more particularly from the remote transmission module 411 integrated into the control device 400, also capable of sending radio frequency configuration signals Sc.

The energy collection means 326 may include the antenna 351 that is also capable of receiving radio frequency configuration signals Sc.

Thus, the control device 400 may also be used to send radio frequency power supply signals Sa to at least partially supply power to the commutation device 300. The energy collection means 326 are capable of receiving these radio frequency power supply signals Sa and for example transferring them into a power supply voltage.

All energy collection means 326 and the external source form a set of remote power supply means capable of at least partially supplying power to the commutation device 300.

The external energy source is not necessarily included in the control device 400. For example, it may be included in the stimulator or it may be independent.

Figure 4 illustrates an example of an electrostimulation device according to the invention including firstly the commutation device 300. This commutation device is provided with a switching circuit 330 with 4 inputs e1, ..., e4 and 20 outputs s1, ..., s20. The inputs e1, ..., e4 are connected to channels v1, ..., v4 of a stimulator 100 transmitting an electrical stimulation signal St on the channel v1. The outputs s1, ..., s20 are connected to the corresponding active areas (202a, 202b, ..., 202t) of several electrodes.

The switching circuit 330 consists of a matrix 331 of 80 switches (ie1,s1, ie2,s1, ie3,s1, ie4,s1, ..., ie1,s20, ie2, s20, ie3, s20, ie4,s20). Each switch may be open or closed. A control circuit 340 connected to the switching circuit 330 can send activation signals to the switches to open or close the switches.

In figure 4, the switch ie1,s1 is closed, and it can thus be used to connect the input e1 with the output s1. The switch ie1,s20 is also closed and connects the input e1 to the output s20. Thus, the switching matrix can route stimulation signals St from the channel v1 of the stimulator connected to the input e1 to two active areas 202a and 202t connected to the outputs s1 and s20 of the commutation device 300 respectively.

The switching circuit 330 is a MEMS. The control circuit 340 may be controlled from an external control device (not shown) using a program or configuration signals informing it which switches in the matrix it should open or close.

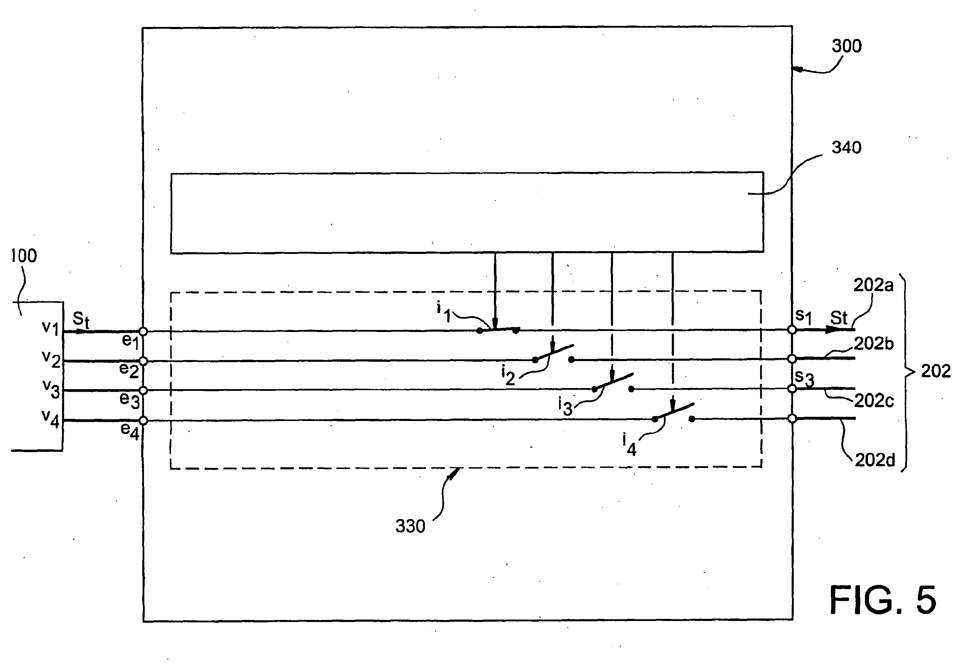

Figure 5 illustrates another example of a device according to the invention in which the commutation device comprises 4 outputs s1, ..., s4 connected to 4 corresponding active areas (202a, 202b, 202c, 202d) of an electrode 202. The switching circuit 330 comprises 4 switches i1, i2, i3, i4, each switch being capable of connecting or not connecting each input e1, ..., e4 of the commutation device with a single output among the outputs s1, s..., s4. The commutation device 300 will be implanted subcutaneously and is connected to the stimulator that sends the electrical stimulation signal St on its 4 channels v1, v2, v3, v4, to the corresponding inputs e1, e..., e4 of the commutation device 300. In figure 5, only switch i1 located between the input e1 and the output s1 is closed. It thus connects the channel v1 of the stimulator 100 to the active area 202a of the electrode 202. As in the device illustrated in figure 4 and described above, the state of each switch can be modified by the control circuit 340, itself controlled by a control device external to the commutation device 300.

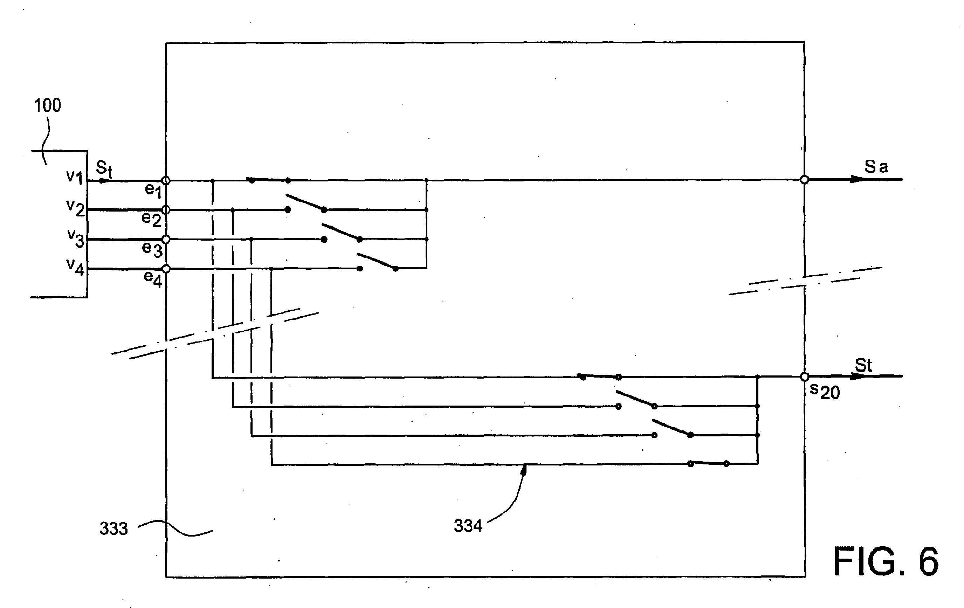

Figure 6 illustrates an example of an electrostimulation device according to the invention including firstly an interconnection device 333 with 4 inputs e1, ..., e4 and 20 outputs s1, ..., s20. The inputs e1, ..., e4 are connected to channels v1, ..., v4 respectively of a stimulator 100, for example including a current generator, emitting an electrical stimulation signal St on the channel v1. The outputs s1, ..., s20 are connected to the corresponding active areas (202a, 202b, ..., 202t) of several electrodes.

The interconnection device 333 is formed from a interconnections matrix 334 that may or may not connect each of the inputs to one or several outputs, in a predetermined manner. Thus, the interconnection matrix can route stimulation signals St, originating in the example illustrated from the stimulator channel v1, towards several predetermined outputs. Any other predetermined and fixed configuration of the matrix could be envisaged.

This type of interconnection circuit 333 can be used to connect one or several channels of a stimulator to one or several target active areas of electrodes without consuming any energy or only consuming a very small quantity of energy due to the fixed nature of the chosen configuration.

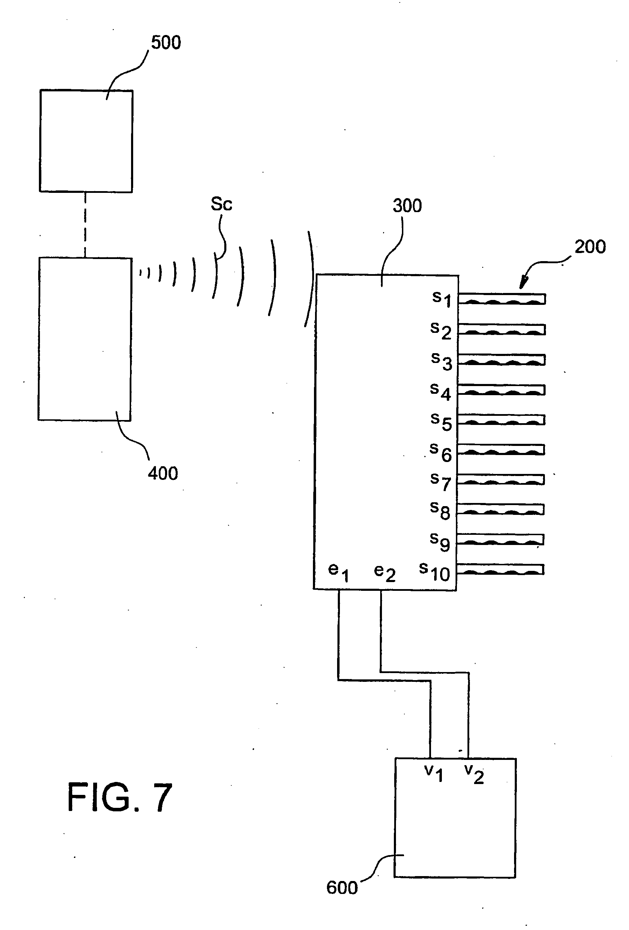

Figure 7 illustrates another example device according to the invention. This device comprises 10 hybrid electrodes each comprising an active area (not shown) that for example is used to make a record of electrical signals identifying a cerebral activity, or to apply stimulation signals in the form of electrical pulses. The electrodes 200 are connected to the corresponding 10 outputs s1, ..., s10 of a commutation device 300. This commutation device has two inputs e1 and e2 connected to the corresponding channels v1 and v2 of a measurement device 600. The switching circuit 300 is used to connect or disconnect the input e1 and /or the input e2, for example to any one of the outputs among outputs s1, ..., s10, or to several outputs among the outputs s1, ..., s10. The measurement device 600 can be used to process electrical signals, for example cerebral activity measurement signals, originating from one or several electrodes when these electrodes are connected by the commutation device 300 to the measurement device 600. The measurement device 600 may also comprise means of sending stimulation pulses to the commutation device 300.

A control device 400 external to the commutation device 300 can be used to control or program the commutation device 300 through configuration signals Sc, and thus to inform the commutation device about which outputs and inputs it should connect.

The control device itself may be programmed by programming means 500.

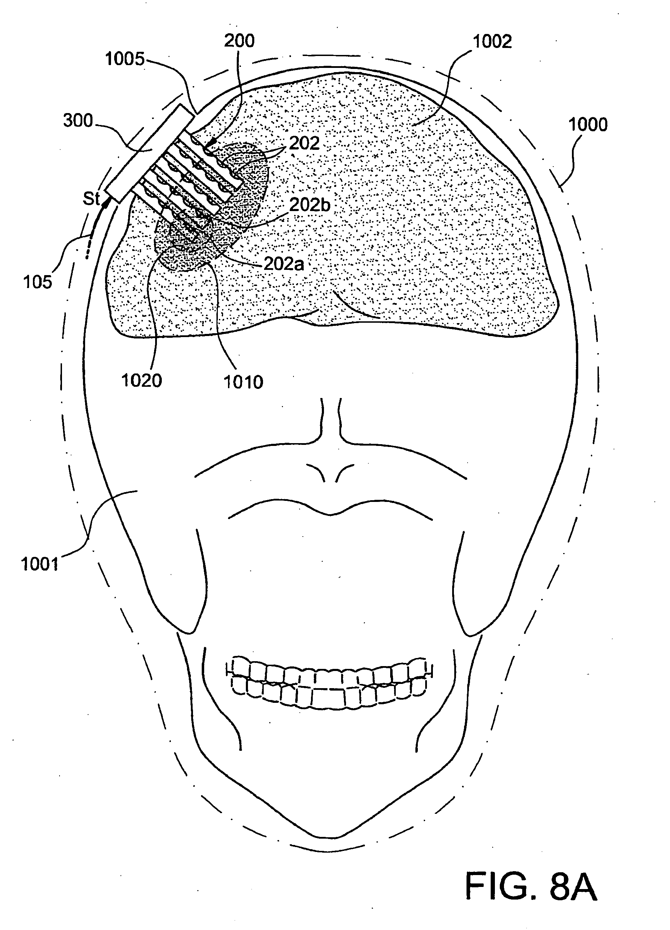

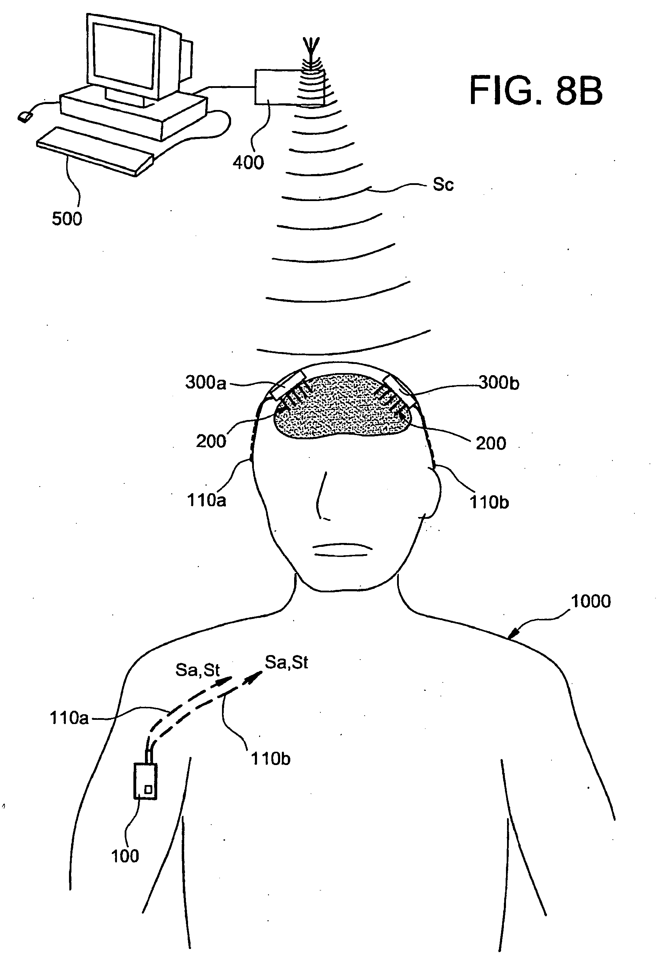

Figures 8A and 8B illustrate a method using an example of an electrostimulation device according to the invention. This method may be applied for processing some cerebral pathologies that can benefit from the input of electrostimulation techniques.

The electrostimulation device may also be used to send electrical pulses to a target region 1020 of a patient's brain 1002 (figure 8A).

Firstly, preferably during an operation performed in an operating theatre, a group of n (for example n = 5) stimulation electrodes 200 each comprising p (for example p = 4) active areas 202, is implanted in openings 1005 made in the patient's skull 1001.

These electrodes 200 are put into contact with the brain 1002 of the patient 1000 on a predetermined region 1010, for example predetermined by a doctor or a surgeon.

This predetermined region 1010 is evaluated by the doctor or the surgeon, as including the target region 1020 to be stimulated in the brain.

The electrodes 200 may be implanted in this region 1010 without very precisely knowing the position of the target region 1020, but for example such that at least one of the active areas of the electrodes at least partially comes into contact with this target region 1020.

According to the invention, it is not compulsory to carry out a test phase to position the target region 1020 before the implantation as was described above according to prior art. Implanting electrodes 200 without performing this test phase reduces the time spent by the patient in the operating theatre. The exact position of the target region can then be determined after the electrodes 200 have been implanted.

The electrodes are connected to a commutation device 300 implanted subcutaneously after the electrodes are implanted, in an area close to the openings 1005 made in the patient's skull 1001.

The commutation device may be in the form of a box, for example with dimensions varying from a few cm² to a few tens of cm².

The box is preferably made from a biocompatible material or a material surrounded by a biocompatible material, for example such as silicone. The commutation device 300 is also used to retransmit electrical stimulation signals St reaching it through a subcutaneous wire 105 originating from a stimulator 100 (shown in figure 8B). These signals may be retransmitted to any one of the active areas 202 of the group of electrodes 200, or to several of them.

Next, once the electrodes 200 have been implanted, the skull may be closed and the commutation device 300 is placed subcutaneously. The patient may then be evacuated from the operating theatre.

The next step is a phase to detect the target region 1020 to be stimulated. Therefore, unlike with techniques known in the past, this detection phase is not necessarily done in the operating theatre.

The resulting saving is measured partly in terms of cost because the operating theatre is no longer exclusively occupied except during the operation to implant the electrodes (which is the least time consuming operation), and partly in psychological terms for the patient himself.

Each active area or group of active areas may be tested in turn, using a commutation device contained in the device according to the invention. Stimulation signals St may be sent in the form of electrical pulses to one or several of the current active areas among the different active areas 202. The patient's reactions to the stimulation are then observed and it is determined whether any of the patient's reactions are symptomatic.

If so, one or several of the current active areas onto which electrical pulses are sent are the target active areas denoted 202a and 202b in figure 8A. These target active areas 202a, 202b correspond to the active areas coming into contact with the target region 1020 to be stimulated.

For example, the device may be used in the treatment of pathologies such as epilepsy or Parkinson's disease. For the treatment of Parkinson's disease, the target region of the brain to be stimulated then generally corresponds to a region of the subthalamic nucleus. When this target region of the subthalamic nucleus is electrically stimulated, the patient's reactions may result in symptomatic reactions stopping uncontrolled movements, or sideration phenomena typically observed in patients suffering from Parkinson's disease.

The commutation device itself is controlled or programmed from outside by a control device 400 integrating a remote transmission device, as already described above. For example, the control device may comprise a headset or a remote control.

According to one variant of the therapeutic method described above, the commutation device 300 like one of those described above and illustrated in one of Figures 1 to 5, may be replaced after one or several hours, or possibly one or several days, or one or several months, by an interconnection device 333 like that described above and illustrated in figure 6. Thus, by experimenting with a patient, a doctor or a surgeon will be able to precisely determine the target region 1020 of the brain of the patient 1000 that he wants to stimulate, using the commutation device 300. If this target region is not likely to change, the initial commutation device 300 can then be replaced by the interconnection device 333 with a fixed configuration, consuming little or no energy, and possibly smaller than the commutation device 300 and that, like the commutation device 300, can be used to connect at least one of the stimulator channels with one or several target active areas of electrodes. In this way, the device comprising the electrodes and the interconnection device may remain implanted in the patient's brain, while consuming little or no energy.

Figure 8B illustrates another example of the method using the electrostimulation device according to the invention. Two groups of n (for example n = 5) electrodes 202, each comprising p (for example p = 4) active areas (not shown) are implanted in the skull of a patient 1000, on each side of the brain. The 2 groups of electrodes are each connected to commutation devices denoted 300a, 300b implanted subcutaneously, each being connected through subcutaneously implanted cables denoted 110a, 110b, to the same stimulator 100. Each cable is placed under the patient's dermis and comprises several wires (not shown). For example, the stimulator sends electrical stimulation signals St on some on the wires and power supply signals Sa on other wires, to supply power to the commutation devices 300. The stimulator is also implanted subcutaneously.

A person, for example a doctor or a surgeon, uses programming means 500, for example a computer with a programming interface and one or several peripherals, to choose one or several target electrodes among the electrodes, or one or several active target areas among the electrode active areas, to which he would like to send electrical pulses. When the choice has been made, the programming means 500 transmit a configuration program or order indicating the choice of target electrodes or target active areas to a control device 400 connected to the programming means 500. The control device then sends radio frequency configuration signals Sc transmitting the configuration program or order to the two commutation devices 300a, 300b. The commutation devices 300a, 300b then make the selection of target electrodes or target active areas chosen by the doctor or the surgeon.

According to one variant of the method described above, the stimulator 100 may be replaced by a measurement device, for example external to the patient. The measurement device then receives electrical measurement signals sent to it from commutation devices 300a and 300b, themselves connected to electrodes that can make the measurements. In the same way as with the method described above, a doctor can use the programming means 500 and the control device 400 to choose one or several target electrodes among the electrodes, or one or several target active areas among the active areas of electrodes, with which he would like to make local measurements of cerebral activities; therefore a device according to the invention will be implanted in the brain during a surgical operation, comprising a step to open the skull, tissues surrounding the brain and to implant several electrodes.

If the commutation device is used for a subcutaneous implantation, it will then be implanted with the electrodes during the same operation.

The surgeon will then immediately close the skull, and the patient will then be evacuated from the operating theatre.

The measurement or test phases can then be performed outside the operating theatre, the commutation device or the switch 300 receiving switching instructions from a control device 400.

This measurement or test step can then be used to determine the open or closed configuration of the various switching means. Once the stimulator 100 has been set up in the switch 300, it can send pulses that will be transmitted to the selected areas of the various electrodes.

It is possible to modify the switching configuration at any time, using the control device 400. This modification does not require any surgical operation.

Like the electrodes 200, the switching means or device 300 may remain implanted in the patient's brain for a very long period, up to several years, and the stimulator 100 can be implanted under the patient's skin, as mentioned above.

According to one variant of the method described above, if the target region to be stimulated is not likely to change, the initial commutation device 300 can then be replaced by the interconnection device 333 illustrated in figure 6 and described above, this device having a fixed configuration and only consuming a very small amount of energy, or no energy.

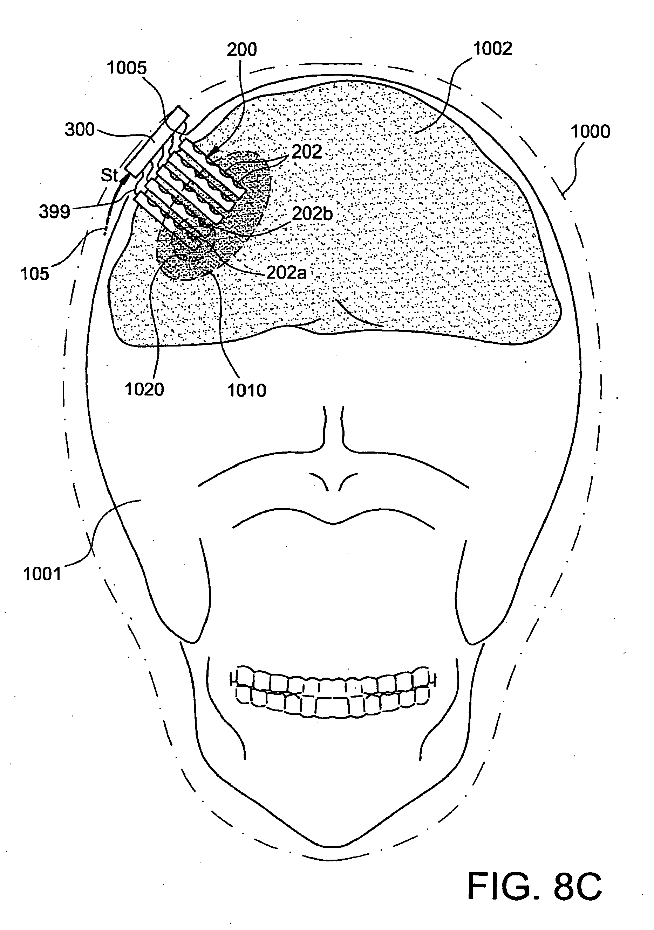

Figure 8C illustrates a variant of the electrostimulation device and is different from figure 8A in that the commutation device 300 and the electrodes 200 are interconnected through flexible cables 399. The electrodes 200 are independent of the switching module 300, and the switching module may be detached from the electrodes and for example replaced by the interconnection device 333 illustrated in figure 6 and described above.

## Claims (Claims for the following Contracting State(s): CH, DE, FR, LI, NL, SE.)

1. Cerebral electrostimulation system containing at least one commutation device (300) or at least one interconnection device (333) comprising:
- switching means
- at least one input and several outputs each connected to at least one biocompatible electrode (200) or at least one active area (202) of a biocompatible electrode (200), the commutation device (300) being used to selectively connect at least one input to one or more outputs or the interconnection device (333) being used to connect each of one or more predetermined fixed inputs to one or more predetermined fixed outputs,
- measurement electrodes,
**Characterized in that:**
- Said switching means comprise electromechanical bistable switches included in a microelectromechanical system,
- and the electrostimulation system further comprises at least one measurement device (600) adapted to process cerebral activity measurement signals.

2. Cerebral electrostimulation system according to claim 1, the commutation device (300) also containing one or more antennas.

3. Cerebral electrostimulation system according to either claim 1 or 2, also containing one control device (400) external to the commutation device (300) capable of controlling or programming the commutation device (300) by radio and / or electrical signals.

4. Cerebral electrostimulation system according to claim 3, the control device (400) containing remote transmission means.

5. Cerebral electrostimulation system according to claim 3, the control device (400) containing remote transmission means to send radio frequency signals Sc.

6. Cerebral electrostimulation system according to one of claims 3 to 5, also containing means (500) capable of programming the control device (400).

7. Cerebral electrostimulation system according to one of claims 1 to 6, also containing power supply means for supplying power to the commutation device (300).

8. Cerebral electrostimulation system according to claim 7, the power supply means including a power supply (321) integrated in the commutation device (300).

9. Cerebral electrostimulation system according to either claim 7 or 8, the power supply means comprising a remote power supply device.

10. Cerebral electrostimulation system according to claim 9, in which the remote transmission device comprises at least one energy source (415) external to the commutation device (300), capable of supplying energy to the commutation device in the form of a radio wave and the system further comprising energy collection means integrated into the commutation device (300) capable of picking up said energy, the energy source (415) being integrated into a control device (200).

11. Cerebral electrostimulation system according to any of claims 1 to 10, comprising at least one stimulator (100).

12. Cerebral electrostimulation system according to claim 11, the stimulator (100) being provided with an integrated power supply (101).

13. Cerebral electrostimulation system according to claim 11 or 12, the stimulator (100) comprising one or more channels connected to one or more inputs of the commutation device (300).

14. Cerebral electrostimulation system according to claim 11 to 13, said stimulator (100) being adapted to send electrical pulses with different amplitudes and/or different frequencies and/or different widths on each input.

15. Cerebral electrostimulation system according to one of claims 11 to 14, said stimulator being adapted to send stimulation signals in the form of electrical pulses, the frequency of the pulses being between 100 Hz and 150 Hz or between 10 kHz and 20 kHz.

16. Cerebral electrostimulation system according to one of claims 1 to 15, comprising at least one measurement device (600) with one or more channels connected to one or more inputs of the commutation device (300).

17. Cerebral electrostimulation system according to one of claims 1 to 16, in which the switching means are arranged in matrix form.

## Claims (Claims for the following Contracting State(s): AT,BE,BG,CY,CZ,DK,EE,ES,FI,GB,GR,HU,IE,IT,LU,MC,PL,PT,RO,SI,SK,TR)

1. Cerebral electrostimulation system containing at least one commutation device (300) or at least one interconnection device (333) comprising:
- switching means,
- at least one input and several outputs each connected to at least one biocompatible electrode (200) or at least one active area (202) of a biocompatible electrode (200), the commutation device (300) being used to selectively connect at least one input to one or more outputs or the interconnection device (333) being used to connect each of one or more predetermined fixed inputs to one or more predetermined fixed outputs,
**characterized in that:**
Said switching means comprise electromechanical bistable switches included in a microelectromechanical system.

2. Cerebral electrostimulation system according to claim 1, the commutation device (300) also containing one or more antennas.

3. Cerebral electrostimulation system according to either claim 1 or 2, also containing one control device (400) external to the commutation device (300) capable of controlling or programming the commutation device (300) by radio and / or electrical signals.

4. Cerebral electrostimulation system according to claim 3, the control device (400) containing remote transmission means.

5. Cerebral electrostimulation system according to claim 3, the control device (400) containing remote transmission means to send radio frequency signals Sc.

6. Cerebral electrostimulation system according to one of claims 3 to 5, also containing means (500) capable of programming the control device (400).

7. Cerebral electrostimulation system according to one of claims 1 to 6, also containing power supply means for supplying power to the commutation device (300).

8. Cerebral electrostimulation system according to claim 7, the power supply means including a power supply (321) integrated in the commutation device (300).

9. Cerebral electrostimulation system according to either claim 7 or 8, the power supply means comprising a remote power supply device.

10. Cerebral electrostimulation system according to claim 9, in which the remote transmission device comprises at least one energy source (415) external to the commutation device (300), capable of supplying energy to the commutation device in the form of a radio wave and the system further comprising energy collection means integrated into the commutation device (300) capable of picking up said energy, the energy source (415) being integrated into a control device (200).

11. Cerebral electrostimulation system according to one of claims 1 to 10, said system comprising stimulation electrodes and / or measurement electrodes and / or a combination of stimulation electrodes and measurement electrodes.

12. Cerebral electrostimulation system according to one of claims 1 to 11, also comprising at least one stimulator (100) and / or one measurement device (600).

13. Cerebral electrostimulation system according to claim 12, comprising at least one stimulator (100) provided with an integrated power supply (101).

14. Cerebral electrostimulation system according to either claim 12 or 13, the stimulator (100) comprising one or more channels connected to one or more inputs of the commutation device (300).

15. Cerebral electrostimulation system according to one of claims 12 to 14, comprising at least one measurement device (600) with one or more channels connected to one or more inputs of the commutation device (300).

16. Cerebral electrostimulation system according to claims 12 to 15, said stimulator (100) being adapted to send electrical pulses with different amplitudes and/or different frequencies and/or different widths on each input.

17. Cerebral electrostimulation system according to one of claims 12 to 16, said stimulator being adapted to send stimulation signals in the form of electrical pulses, the frequency of the pulses being between 100 Hz and 150 Hz or between 10 kHz and 20 kHz.

18. Cerebral electrostimulation system according to one of claims 1 to 17, in which the switching means are arranged in matrix form.

## Patentansprüche (Patentansprüche für folgende(n) Vertragsstaat(en): CH,DE,FR,LI,NL,SE.)

1. Gehirn-Elektrostimulationssystem, das wenigstens eine Kommutierungsvorrichtung (300) oder wenigstens eine Zusammenschaltungsvorrichtung (333) enthält, wobei das System umfasst:
- Schalteinrichtungen,
- wenigstens einen Eingang und mehrere Ausgänge, jeder mit wenigstens einer biokompatiblen Elektrode (200) oder mit wenigstens einer Aktivfläche (202) einer biokompatiblen Elektrode (200) verbunden, wobei die Kommutierungsvorrichtung (300) verwendet wird, um selektiv wenigstens einen Eingang mit einem oder mehreren Ausgängen zu verbinden oder die Zusammenschaltungsvorrichtung (333) verwendet wird, um jeden von einem oder mehreren vorgegebenen feststehenden Eingängen mit einem oder mehreren vorgegebenen feststehenden Ausgängen zu verbinden, und
- Messelektroden,
**dadurch gekennzeichnet, dass**
die Schalteinrichtungen elektromechanische bistabile Schalter umfassen, die in einem mikroelektromechanischen System enthalten sind,
und das Elektrostimulationssystem des Weiteren wenigstens eine Messeinrichtung (600) umfasst, die eingerichtet ist, um Messsignale der Gehirnaktivität zu verarbeiten.

2. Gehirn-Elektrostimulationssystem nach Anspruch 1, wobei die Kommutierungsvorrichtung (300) außerdem eine oder mehrere Antennen enthält.

3. Gehirn-Elektrostimulationssystem nach Anspruch 1 oder 2, das außerdem eine Steuereinrichtung (400) enthält, die extern zu der Kommutierungsvorrichtung (300) ist und fähig zum Steuern oder Programmieren der Kommutierungsvorrichtung (300) durch Funksignale und/oder elektrische Signale ist.

4. Gehirn-Elektrostimulationssystem nach Anspruch 3, wobei die Steuereinrichtung (400) Fernübertragungseinrichtungen enthält.

5. Gehirn-Elektrostimulationssystem nach Anspruch 3, wobei die Steuereinrichtung (400) Fernübertragungseinrichtungen enthält, um Funkfrequenzsignale Sc zu senden.

6. Gehirn-Elektrostimulationssystem nach einem der Ansprüche 3 bis 5, das außerdem Einrichtungen (500) enthält, die fähig zum Programmieren der Steuereinrichtung (400) sind.

7. Gehirn-Elektrostimulationssystem nach einem der Ansprüche 1 bis 6, das außerdem Stromversorgungseinrichtungen zum Versorgen der Kommutierungsvorrichtung (300) mit Strom enthält.

8. Gehirn-Elektrostimulationssystem nach Anspruch 7, wobei die Stromversorgungsvorrichtungen eine in die Kommutierungsvorrichtung (300) integrierte Stromversorgung (321) einschließen.

9. Gehirn-Elektrostimulationssystem nach Anspruch 7 oder 8, wobei die Stromversorgungseinrichtungen eine Fernstromversorgungsvorrichtung umfassen.

10. Gehirn-Elektrostimulationssystem nach Anspruch 9, wobei die Fernübertragungsvorrichtung wenigstens eine Energiequelle (415) umfasst, die extern zu der Kommutierungsvorrichtung (300) ist und fähig zum Versorgen der Kommutierungsvorrichtung mit Energie in Form einer Funkwelle ist, und das System des Weiteren eine in die Kommutierungsvorrichtung (300) integrierte Energie-Sammeleinrichtung umfasst, die fähig zum Aufnehmen der Energie ist, wobei die Energiequelle (415) in eine Steuervorrichtung (200) integriert ist.

11. Gehirn-Elektrostimulationssystem nach einem der Ansprüche 1 bis 10, das außerdem wenigstens einen Stimulator (100) umfasst.

12. Gehirn-Elektrostimulationssystem nach einem der Ansprüche 11, das wenigstens einen Stimulator (100) umfasst, der mit einer integrierten Stromversorgung (101) versehen ist.

13. Gehirn-Elektrostimulationssystem nach Anspruch 11 oder 12, wobei der Stimulator (100) eine oder mehrere Kanäle umfasst, die mit einem oder mehreren Eingängen der Kommutierungsvorrichtung (300) verbunden sind.

14. Gehirn-Elektrostimulationssystem nach Anspruch 11 bis 13, wobei der Stimulator (100) eingerichtet ist, um elektrische Impulse mit verschiedenen Amplituden und/oder verschiedenen Frequenzen und/oder verschiedenen Breiten an jeden Eingang zu senden.

15. Gehirn-Elektrostimulationssystem nach einem der Ansprüche 11 bis 14, wobei der Stimulator eingerichtet ist, um Stimulationssignale in Form elektrischer Impulse zu senden, wobei die Frequenz der Impulse zwischen 100 Hz und 150 Hz oder zwischen 10 kHz und 20 kHz ist.

16. Gehirn-Elektrostimulationssystem nach Anspruch 1 bis 15, das wenigstens eine Messeinrichtung (600) mit einem oder mehreren Kanälen mit einem oder mehreren Ausgängen der Kommutierungsvorrichtung (300) verbunden umfasst.

17. Gehirn-Elektrostimulationssystem nach Anspruch 1 bis 16, wobei die Schalteinrichtungen in Matrixform eingerichtet sind.

## Patentansprüche (Patentansprüche für folgende(n) Vertragsstaat(en): AT,BE,BG,CY,CZ,DK,EE,ES,FI,GB,GR,HU,IE,IT,LU,MC,PL,PT,RO,SI,SK,TR)

1. Gehirn-Elektrostimulationssystem, das wenigstens eine Kommutierungsvorrichtung (300) oder wenigstens eine Zusammenschaltungsvorrichtung (333) enthält, wobei das System umfasst:
- Schalteinrichtungen,
- wenigstens einen Eingang und mehrere Ausgänge, jeder mit wenigstens einer biokompatiblen Elektrode (200) oder mit wenigstens einer Aktivfläche (202) einer biokompatiblen Elektrode (200) verbunden, wobei die Kommutierungsvorrichtung (300) verwendet wird, um selektiv wenigstens einen Eingang mit einem oder mehreren Ausgängen zu verbinden oder die Zusammenschaltungsvorrichtung (333) verwendet wird, um jeden von einem oder mehreren vorgegebenen feststehenden Eingängen mit einem oder mehreren vorgegebenen feststehenden Ausgängen zu verbinden,
**dadurch gekennzeichnet, dass**
die Schalteinrichtungen elektromechanische bistabile Schalter umfassen, die in einem mikroelektromechanischen System enthalten sind.

2. Gehirn-Elektrostimulationssystem nach Anspruch 1, wobei die Kommutierungsvorrichtung (300) außerdem eine oder mehrere Antennen enthält.

3. Gehirn-Elektrostimulationssystem nach Anspruch 1 oder 2, das außerdem eine Steuereinrichtung (400) enthält, die extern zu der Kommutierungsvorrichtung (300) ist und fähig zum Steuern oder Programmieren der Kommutierungsvorrichtung (300) durch Funksignale und/oder elektrische Signale ist.

4. Gehirn-Elektrostimulationssystem nach Anspruch 3, wobei die Steuereinrichtung (400) Fernübertragungseinrichtungen enthält.

5. Gehirn-Elektrostimulationssystem nach Anspruch 3, wobei die Steuereinrichtung (400) Fernübertragungseinrichtungen enthält, um Funkfrequenzsignale Sc zu senden.

6. Gehirn-Elektrostimulationssystem nach einem der Ansprüche 3 bis 5, das außerdem Einrichtungen (500) enthält, die fähig zum Programmieren der Steuereinrichtung (400) sind.

7. Gehirn-Elektrostimulationssystem nach einem der Ansprüche 1 bis 6, das außerdem Stromversorgungseinrichtungen zum Versorgen der Kommutierungsvorrichtung (300) mit Strom enthält.

8. Gehirn-Elektrostimulationssystem nach Anspruch 7, wobei die Stromversorgungsvorrichtungen eine in die Kommutierungsvorrichtung (300) integrierte Stromversorgung (321) einschließen.

9. Gehirn-Elektrostimulationssystem nach Anspruch 7 oder 8, wobei die Stromversorgungseinrichtungen eine Fernstromversorgungsvorrichtung umfassen.

10. Gehirn-Elektrostimulationssystem nach Anspruch 9, wobei die Fernübertragungsvorrichtung wenigstens eine Energiequelle (415) umfasst, die extern zu der Kommutierungsvorrichtung (300) ist und fähig zum Versorgen der Kommutierungsvorrichtung mit Energie in Form einer Funkwelle ist, und das System des Weiteren eine in die Kommutierungsvorrichtung (300) integrierte Energie-Sammeleinrichtung umfasst, die fähig zum Aufnehmen der Energie ist, wobei die Energiequelle (415) in eine Steuervorrichtung (200) integriert ist.

11. Gehirn-Elektrostimulationssystem nach einem der Ansprüche 1 bis 10, wobei das System Stimulationselektroden und/oder Messelektroden und/oder eine Kombination von Stimulationselektroden und Messelektroden umfasst.

12. Gehirn-Elektrostimulationssystem nach einem der Ansprüche 1 bis 11, das außerdem wenigstens einen Stimulator (100) und/oder eine Messeinrichtung (600) umfasst.

13. Gehirn-Elektrostimulationssystem nach Anspruch 12, das wenigstens einen Stimulator (100) umfasst, der mit einer integrierten Stromversorgung (101) versehen ist.

14. Gehirn-Elektrostimulationssystem nach Anspruch 12 oder 13, wobei der Stimulator (100) eine oder mehrere Kanäle umfasst, die mit einem oder mehreren Eingängen der Kommutierungsvorrichtung (300) verbunden sind.

15. Gehirn-Elektrostimulationssystem nach einem der Ansprüche 12 bis 14, das wenigstens eine Messeinrichtung (600) mit einem oder mehreren Kanälen mit einem oder mehreren Ausgängen der Kommutierungsvorrichtung (300) verbunden umfasst.

16. Gehirn-Elektrostimulationssystem nach Anspruch 12 bis 15, wobei der Stimulator (100) eingerichtet ist, um elektrische Impulse mit verschiedenen Amplituden und/oder verschiedenen Frequenzen und/oder verschiedenen Breiten an jeden Eingang zu senden.

17. Gehirn-Elektrostimulationssystem nach einem der Ansprüche 12 bis 15, wobei der Stimulator eingerichtet ist, um Stimulationssignale in Form elektrischer Impulse zu senden, wobei die Frequenz der Impulse zwischen 100 Hz und 150 Hz oder zwischen 10 kHz und 20 kHz ist.

18. Gehirn-Elektrostimulationssystem nach Anspruch 1 bis 17, wobei die Schalteinrichtungen in Matrixform eingerichtet sind.

## Revendications (Revendications pour l'(les) Etat(s) contractant(s) suivant(s): CH, DE, FR, LI, NL, SE.)

1. Système d'électrostimulation cérébrale comprenant au moïns un dispositif de commutation (300) ou au moins un dispositif d'interconnexion (333) comportant :
- des moyens interrupteurs,
- au moins une entrée et plusieurs sorties chacune reliée avec au moins une électrode (200) biocompatible ou au moins une zone active (202) d'une électrode (200) biocompatible, le dispositif de commutation (300) permettant de connecter sélectivement au moins une entrée fixe prédéterminée(s) à une ou plusieurs sorties fixes prédéterminée(s), ou le dispositif d'interconnexion (333) permettant de connecter sélectivement au moins une entrée fixe prédéterminée(s) à une ou plusieurs sorties fixes prédéterminée(s),
- des électrodes de mesure,
**caractérisé en ce que :**
- lesdits moyens interrupteurs comprennent des bistables électromécaniques compris dans un microsystème électromécanique,
- ledit dispositif d'électrostimulation comprenant en outre au moins un dispositif de mesure (600) adapté au traitement de signaux de mesure d'activité cérébrale.

2. Système d'électrostimulation cérébrale selon la revendication 1, le dispositif de commutation (300) comprenant en outre une ou plusieurs antennes.

3. Système d'électrostimulation cérébrale selon l'une des revendications 1 ou 2, comprenant en outre un dispositif de commande (400) extérieur au dispositif de commutation (300) apte à piloter ou programmer le dispositif de commutation (300) par des signaux radio ou/et électriques.

4. Système d'électrostimulation cérébrale selon la revendication 3, le dispositif de commande (400) comprenant des moyens de télétransmission.

5. Système d'électrostimulation cérébrale selon la revendication 3, le dispositif de commande (400) comprenant des moyens de télétransmission pour envoyer des signaux radiofréquence Sc.

6. Système d'électrostimulation cérébrale selon l'une des revendications 3 à 5, comprenant en outre : des moyens de programmation (500) aptes à programmer le dispositif de commande (400).

7. Système d'électrostimulation cérébrale selon l'une des revendications 1 à 6, comprenant en outre des moyens d'alimentation aptes à alimenter le dispositif de commutation (300).

8. Système d'électrostimulation cérébrale selon la revendication 7, les moyens d'alimentation comportant une alimentation intégrée (321) au dispositif de commutation (300).

9. Système d'électrostimulation cérébrale selon l'une des revendications 7 ou 8, les moyens d'alimentation comprenant un dispositif de téléalimentation.

10. Système d'électrostimulation cérébrale selon la revendication 9, dans lequel le dispositif de téléalimentation comprend au moins une source d'énergie (415) extérieure au dispositif de commutation (300), apte à émettre une énergie sous forme d'une onde radio, le système comprenant en outre des moyens capteurs d'énergie intégrés dans le dispositif de commutation (300) aptes à capter ladite énergie, la source d'énergie (415) étant intégrée au dispositif de commande (200).

11. Dispositif d'électrostimulation cérébrale selon l'une des revendication 1 à 10, comprenant en outre : au moins un stimulateur (100).

12. Système d'électrostimulation cérébrale selon la revendication 11, comprenant au moins un stimulateur (100) doté d'une alimentation intégrée (101).

13. Système d'électrostimulation cérébrale selon la revendication 11 ou 12, le stimulateur (100) comportant une ou plusieurs voies reliées à une ou plusieurs entrées du dispositif de commutation (300).

14. Dispositif d'électrostimulation cérébrale selon l'une des revendications 11 à 13, ledit stimulateur étant adapté pour envoyer des impulsions électriques d'amplitudes différentes et/ou de fréquences différentes et/ou de largeurs différentes sur chaque entrée.

15. Dispositif d'électrostimulation cérébrale selon l'une des revendications 11 à 14, ledit stimulateur étant adapté pour envoyer des signaux de stimulation sous forme d'impulsions électriques, la fréquence des impulsions électriques étant comprise entre 100 Hz et 150 Hz ou entre 10 kHz et 20 kHz.

16. Dispositif d'électrostimulation cérébrale selon l'une des revendications 1 à 15, le dispositif de mesure (600), comportant une ou plusieurs voies reliées à une ou plusieurs entrées du dispositif de commutation (300).

17. Dispositif d'électrostimulation cérébrale selon l'une des revendications 1 à 16, dans lequel les moyens interrupteurs sont agencés selon une matrice.

## Revendications (Revendications pour l'(les) Etat(s) contractant(s) suivant(s): AT,BE,BG,CY,CZ,DK,EE,ES,EI,GB,GR,HU,IE,IT,LU,MC,PL,PT,RO,SI,SK,TR)

1. Système d'électrostimulation cérébrale comprenant au moins un dispositif de commutation (300) ou au moins un dispositif d'interconnexion (333) comportant :
- des moyens interrupteurs,
- au moins une entrée et plusieurs sorties chacune reliée avec au moins une électrode (200) biocompatible ou au moins une zone active (202) d'une électrode (200) biocompatible, le dispositif de commutation (300) permettant de connecter sélectivement au moins une entrée fixe prédéterminée(s) à une ou plusieurs sorties fixes prédéterminée(s), ou le dispositif d'interconnexion (333) permettant de connecter sélectivement au moins une entrée fixe prédéterminée(s) à une ou plusieurs sorties fixes prédéterminée(s),
**caractérisé en ce que :**
- lesdits moyens interrupteurs comprennent des bistables électromécaniques compris dans un microsystème électromécanique.

2. Système d'électrostimulation cérébrale selon la revendication 1, le dispositif de commutation (300) comprenant en outre une ou plusieurs antennes.

3. Système d'électrostimulation cérébrale selon l'une des revendications 1 ou 2, comprenant en outre un dispositif de commande (400) extérieur au dispositif de commutation (300) apte à piloter ou programmer le dispositif de commutation (300) par des signaux radio ou/et électriques.

4. Système d'électrostimulation cérébrale selon la revendication 3, le dispositif de commande (400) comprenant des moyens de télétransmission.

5. Système d'électrostimulation cérébrale selon la revendication 3, dans lequel le dispositif de commande (400) est apte à piloter ou programmer le dispositif de commutation (300) via des signaux S_{c} radiofréquence.

6. Système d'électrostimulation cérébrale selon l'une des revendications 3 à 5, comprenant en outre : des moyens de programmation (500) aptes à programmer le dispositif de commande (400).

7. Système d'électrostimulation cérébrale selon l'une des revendications 1 à 6, comprenant en outre des moyens d'alimentation aptes à alimenter le dispositif de commutation (300).

8. Système d'électrostimulation cérébrale selon la revendication 7, les moyens d'alimentation comportant une alimentation intégrée (321) au dispositif de commutation (300).

9. Système d'électrostimulation cérébrale selon l'une des revendications 7 ou 8, les moyens d'alimentation comprenant un dispositif de téléalimentation.

10. Système d'électrostimulation cérébrale selon la revendication 9, dans lequel le dispositif de téléalimentation comprend au moins une source d'énergie (415) extérieure au dispositif de commutation (300), apte à émettre une énergie sous forme d'une onde radio, le système comprenant en outre des moyens capteurs d'énergie intégrés dans le dispositif de commutation (300) aptes à capter ladite énergie, la source d'énergie (415) étant intégrée au dispositif de commande (200).

11. Dispositif d'électrostimulation cérébrale selon l'une des revendications 1 à 10, le dispositif d'électrostimulation comprenant des électrodes de stimulation ou/et des électrodes de mesure ou/et des électrodes à la fois de stimulation et de mesure.

12. Dispositif d'électrostimulation cérébrale selon l'une des revendications 1 à 11, comprenant en outre : au moins un stimulateur (100) ou/et un dispositif de mesure (600).

13. Dispositif d'électrostimulation cérébrale selon la revendication 12, comprenant au moins un stimulateur (100) doté d'une alimentation intégrée (101).

14. Système d'électrostimulation cérébrale selon la revendication 12 ou 13, le stimulateur (100) comportant une ou plusieurs voies reliées à une ou plusieurs entrées du dispositif de commutation (300).

15. Système d'électrostimulation cérébrale selon l'une des revendications 12 à 14, le dispositif de mesure (600) comportant une ou plusieurs voies reliées à une ou plusieurs entrées du dispositif de commutation (300).

16. Dispositif d'électrostimulation cérébrale selon l'une des revendications 12 à 15, ledit stimulateur étant adapté pour envoyer des impulsions électriques d'amplitudes différentes et/ou de fréquences différentes et/ou de largeurs différentes sur chaque entrée.

17. Dispositif d'électrostimulation cérébrale selon l'une des revendications 12 à 16, ledit stimulateur étant adapté pour envoyer des signaux de stimulation sous forme d'impulsions électriques, la fréquence des impulsions électriques étant comprise entre 100 Hz et 150 Hz ou entre 10 kHz et 20 kHz.

18. Dispositif d'électrostimulation cérébrale selon l'une des revendications 1 à 17, dans lequel les moyens interrupteurs sont agencés selon une matrice.
